# EUROPEAN PATENT SPECIFICATION

(11) **EP 3 706 864 B1**
(45) Date of publication and mention of the grant of the patent: **17.03.2021**
(21) Application number: 18829411.0
(22) Date of filing: 26.12.2018
(51) Int. Cl.: A61N 5/10, A61B 34/20

(54) **INVIVO DOSIMETER POSITIONING USING CATHETER RECONSTRUCTION**
IN-VIVO-DOSIMETERPOSITIONIERUNG MITTELS KATHETERREKONSTRUKTION
POSITIONNEMENT DE DOSIMÈTRE IN VIVO AU MOYEN D'UNE RECONSTRUCTION DE CATHÉTER

(30) Priority: 05.01.2018 EP 18150451
(43) Date of publication of application: 16.09.2020
(73) Proprietor: Koninklijke Philips N.V., 5656 AE Eindhoven (NL)
(72) Inventor: HAUTVAST, Guillaume, Leopold, Theodorus, Frederik, 5656 AE Eindhoven (NL); BINNEKAMP, Dirk, 5656 AE Eindhoven (NL)
(74) Representative: Philips Intellectual Property & Standards
(86) International application number: PCT/EP2018/086871
(87) International publication number: WO 2019/134877

(56) References cited:
- EP-A1- 1 616 598
- WO-A1-03/062855
- WO-A1-2011/080606

## Description

### FIELD OF THE INVENTION

The present invention relates to a system for brachytherapy treatment, in particular High Dose Rate (HDR) brachytherapy treatment, employing Invivo Dosimetry (ID) during the treatment process, a quality assurance method and respective computer program. More specifically, the invention relates to a system for accurate positioning of an ID measurement device on the basis of a reconstruction of a guiding tube, in particular a catheter, through which the ID measurement device may be guided to the desired measurement position.

### BACKGROUND OF THE INVENTION

In brachytherapy treatments, growths like cancer or tumors are treated by delivering radiation to a target area. Hereby, a source of radioactive radiation is typically moved through implanted guiding tubes, such as catheters or channels, inside the patient and hereby irradiates the target area invivo. Typically, the movement of the source of radioactive radiation is achieved using a so-called afterloader, usually controlled by a therapy control system (TCS), which drives the source of radioactive radiation through the respective guiding tube.

In order to achieve good treatment results, it is prudent that the radioactive radiation is appropriately delivered to the target area, i.e. that the amount of radioactive radiation delivered to the target area corresponds to the one anticipated by the therapy plan.

However, in current clinical approaches, the source of radioactive radiation is typically merely moved to a planned position inside the catheter and it is assumed that the irradiation of the target area occurs as planned. Hereby, the radiation dose that has actually been delivered to the target area cannot be verified, but is rather assumed to be equal to the dose anticipated by the treatment plan. This assumption may not always be true due to several potential error sources, such as imprecise positioning of the sources inside the catheter, irregularities in the medium absorbing the dose, movement of the source due to catheter motion or the like. Accordingly, quality assurance (QA) of the dose delivery process is rather limited.

With the introduction of Invivo Dosimetry (ID) a more extensive QA process has become possible. ID involves placing miniature sensors (ID devices) near the target areas. These miniature sensors measure the radioactive dose or the radioactive dose rate during radiation delivery. QA then comprises comparing those measured dose (rates) with the one anticipated by the therapy plan. Accordingly, the introduction of ID allows to verify the dose delivery process and to more accurately reconstruct the actually delivered dose.

However, the delivered dose often may exhibit high dose gradients from one position to another. Accordingly, knowledge about the relative position of the ID devices with respect to the sources of radioactive radiation is crucial for the accuracy of the dose measurement, as small errors in the relative positions may result in large deviations in the dose rates determined by the ID device. The positioning of an ID device may be determined using a tracking method such as electromagnetic (EM) tracking, whereby the ID device is equipped with a tracking sensor and its position is tracked during introduction and positioning.

However, in order to successfully perform said tracking, not only the ID device but also the catheter into which the source is inserted should be tracked, which largely increases the complexity of the QA process. Further, it is not known whether and, if so, how the EM tracking sensors are affected by the source of radioactive radiation during the measurement. Thus, potential EM disturbances introduce a further element of inaccuracy into the QA process.

WO 2011/080606 discloses a system wherein the shape of brachytherapy channels is tracked. Alternatively, radioactive sources are tracked directly. Insertable radioactivity sensors are not mentioned.

EP 1 616 598 discloses a system wherein the distance between the insertable sensor and the radioactive source is calculated based on sensed radiation intensity. The distance is used to estimate balloon inflation.

In the system discussed by WO 03/062855 the sensors are located on additional sensing needles; their signals are used to calculate source positions.

### SUMMARY OF THE INVENTION

As described herein above, the QA process in brachytherapy is currently fairly error-prone. It is therefore an object of the present invention to provide an improved system for brachytherapy treatment which allows for a more accurate QA process. It is a further object to provide a system and a corresponding method which allows to accurately determine the position of the ID devices relative to the sources of radioactive radiation. It is an even further object to optimize the measurement position of the ID devices in a manner that allows a more accurate determination of the dose that is actually delivered to the target area.

The invention is defined in the following claims; other embodiments, examples or therapeutic methods do not constitute parts of the invention.

This objective is achieved by a system for brachytherapy treatment comprising an input unit for receiving tracking data comprising a plurality of tracking values acquired at a plurality of positions along at least one guiding tube and a reconstruction unit for obtaining a reconstruction of said at least one guiding tube based on the tracking data. The system further comprises a determination unit for determining, based on the reconstruction, at least one measurement position of at least one measurement device guided by said at least one guiding tube relative to at least one source position of at least one source of radioactive radiation.

Preferably, the system for brachytherapy also comprises a tracking device configured for acquiring tracking values at a plurality of positions along at least one guiding tube. When the system for brachytherapy is in use, it preferably also comprises the guiding tube and the measurement device and a source of radioactive radiation. However, the guiding tube, measurement device and source are preferably disposables or used in a single patient and are therefore no essential elements of the system for brachytherapy.

In this context, the term brachytherapy treatment may generally refer to the treatment of growths, such as tumors or cancerous growths, by positioning a source of radioactive radiation inside a patient such that the source may irradiate the growth invivo. More particularly, the term brachytherapy treatment may refer to anyone of low dose rate (LDR) brachytherapy, pulsed dose rate (PDR) brachytherapy, high dose rate (HDR) brachytherapy or a combination of those.

Hereby, HDR brachytherapy treatment comprises the insertion of one or more guiding tubes for guiding the sources of radioactive radiation into a patient. Subsequently, the sources of radioactive radiation are positioned, using the guiding tube, at a specific position at which they may sufficiently irradiate the target area. This specific position may also be referred to as the source location. The positioning is typically achieved by attaching the sources of radioactive radiation to so-called drive wires configured for driving through the guiding tubes and by driving the drive wires having the sources attached thereto through the guiding tubes such that the sources are positioned at their respective source location.

In HDR brachytherapy, the sources exhibit an activity of above 12 Gray per hour. As such, typical sources used for HDR brachytherapy are highly radioactive sources such as Iod-125 or Iridium-192 or the like. In contrast to HDR brachytherapy, LDR brachytherapy treatment employs sources exhibiting less activity, typically around 2 Gray per hour. In LDR brachytherapy, the sources are deposited at a fixed position inside the patient's body for an extended time period (usually up to 24 hours). Finally, PDR brachytherapy treatment may be performed as, both, HDR or LDR brachytherapy treatment. During PDR brachytherapy, however, radiation is delivered in pulses.

Brachytherapy may be performed by placing the sources at the source location in the target area temporarily or permanently. In temporary brachytherapy, the sources are placed inside the patient for a pre-set time duration (minutes for HDR brachytherapy, hours for LDR brachytherapy) before being extracted again. The precise placement period is hereby heavily influenced by a variety of parameters, such as the required rate of dose delivery and the type, size and location of the cancer.

On the other hand, permanent brachytherapy - also referred to as seed implantation - may be performed. This kind of brachytherapy involves the placement of sources of radioactive radiation in the form of small LDR radioactive seeds (or pellets) inside the tumor or target area. The seed is maintained at this position where it gradually decays. The seed remains inside the target area for a period of weeks up to months and the radiation emitted by the seed will slowly decrease in accordance with the half-live period of the seed used.

The term target area particularly refers to the area, or region of interest (ROI), that is to be irradiated by the source of radioactive radiation. As such, the target area typically denotes the area of the tumor and/or cancerous growth to be treated. The source of radioactive radiation is hereby positioned in such a manner that the necessary radiation dose impinges on the target area.

In this context, the term guiding tube may particularly refer to a catheter, a channel or a needle that is inserted into the patient and used to guide the source of radioactive radiation and/or the measurement device to an appropriate position. It shall be understood that each guiding tube may be configured to guide precisely one component, i.e. to guide either a source or a measurement device. The insertion of two components into a single guiding tube at the same time should be avoided.

Hereby, these guiding tubes may particularly be implemented as dedicated guiding tubes that are used either exclusively for sources or exclusively for measurement devices. In these cases, the measurement guiding tubes may typically not be suitable to guide sources (e.g. because they are too large or too small) and vice versa. In this context, guiding tubes that are used exclusively for sources shall be denoted source guiding tubes and guiding tubes that are used for measurement devices only shall be denoted measurement guiding tubes. Alternatively or additionally, hybrid guiding tubes may be implemented in the system and each one of these hybrid guiding tubes may be suitable for guiding both, sources and measurement devices, whichever is desired for a particular brachytherapy session.

The term measurement device may typically refer to a miniature sensor for Invivo Dosimetry (ID) capable of measuring radiation data occurring at a particular location inside the patient in the proximity of the measurement device.

Hereby, the measurement devices may particularly be implemented by one or more of the following technologies:
- Metal oxide semiconductor field effect transistors (MOSFETs).
- Thermoluminescent dosimeters (TLDs).
- Plastic scintillation dosimeters.
- Diamond detectors.
- Radiochromic dosimetry material.

The term tracking data particularly refers to a plurality of tracking values obtained at a plurality of positions along a longitudinal axis of the guiding tube used for guiding the measurement device. It shall be understood that this guiding tube may either be a measurement guiding tube or a hybrid guiding tube.

In order to obtain such tracking values, at least one tracking device can be attached to a drive wire that is then driven through the respective guiding tube and allows to collect respective tracking values at each of the plurality of positions. The tracking data obtained for the plurality of positions can then be used to reconstruct the tracked measurement guiding tube. Hereby, the term reconstruction may particularly refer to a determination of the shape and course of the measurement guiding tube inside the patient from the tracking data. Such as reconstruction of a guiding tube from tracking data is disclosed in WO2009156893 in relation to guiding tubes that are used to position sources of radioactive radiation. Using said tracking data, an appropriate reconstruction of the guiding tubes may be achieved if the tracking process itself is accurate enough.

Based on the reconstruction of the guiding tube that shall guide the measurement device, at least one measurement position for placing the measurement device may be derived. For the sake of further explanation, the guiding tube to guide the measurement device shall be referred to as measurement guiding tube and the guiding tube to guide the source of radioactive radiation shall be designated the source guiding tube. It shall be understood, though, that, whenever the measurement respectively source guiding tube is mentioned, the measurement respectively source guiding tube may be a dedicated guiding tube or a hybrid guiding tube.

A measurement position for the measurement device may be determined from the reconstruction of the guiding tube based on the positional relationship of the measurement guiding tube to the source guiding tube used for guiding the corresponding source of radioactive radiation (i.e. the source which radiation dose is supposed to be measured using the respective measurement device). This allows to determine the positional relationship of the measurement device and the source of radioactive radiation to one another as well. This allows to estimate the dose that should be measured by the measurement device once the source irradiates the target area.

In order to achieve appropriate positioning for the measurement device in the measurement guiding tube and the source in the source guiding tube relative to one another, the positional relationship of the measurement guiding tube and its corresponding source guiding tube have to be correlated to one another.

In some embodiments, this may be achieved by using the same tracking system to obtain the tracking data for the measurement guiding tube and the source guiding tube, respectively. This is particularly achieved in embodiments employing hybrid guiding tubes, in which the reconstruction of the guiding tubes may be performed independent of whether or not they are used for a source or a measurement device later one.

Basing the reconstruction of the measurement guiding tube and the source guiding tube on tracking data obtained with the same tracking system intrinsically defines the positions in their respective reconstructions in the same coordinate system. This allows to directly derive the positional relationship between the measurement guiding tube and the source guiding tube and, therefore, the measurement position relative to the position of the source of radioactive radiation.

In some embodiments, different tracking systems may be used to obtain the tracking data for the measurement guiding tube and its corresponding source guiding tube, respectively. In this case, the reconstructions based on the tracking data are provided in two different coordinate systems and a calibration (or co-registration) between the two tracking modalities and the two coordinate systems is necessary to determine the positional relationship between the measurement guiding tubes and the source guiding tubes. How such a calibration may be performed largely depends on the tracking modality used and, thus, is readily apparent to the person skilled in the relevant art.

As such, the measurement position determined for the measurement device corresponds to a relative position, i.e. a position that has to be determined in relation to a respective source of radioactive radiation and/or the target area to which the radiation shall be delivered. In other words: contrary to the positioning of the sources of radioactive radiation performed on the basis of a reconstruction of the guiding tube as disclosed in WO2009156893, the positioning of the measurement devices has to be performed by determining the (relative) measurement position in view of the source position and/or the target area.

In that context, it has to be understood that, in all cases where the present approach is applied, at least a further source guiding tube for guiding the source of radioactive radiation will be present. As indicated herein above, though, it is not necessary that both guiding tubes have be reconstructed from tracking data of the same tracking system. They may also be reconstructed from tracking data of different tracking systems or it may even be possible to determine the positions along the longitudinal axis of the source guiding tube by different means, as long as these means allow to determine the positional relationship between the measurement guiding tube and the source guiding tube, and, thus, the measurement device and the source of radioactive radiation.

The relative measurement position may then be input - manually or automatically - into a respective control unit configured to position the measurement device. The control unit drives the measurement device through its measurement guide wire to the determined measurement position. Hereby, the control unit may particularly use the positions at which the tracking values in the tracking data have be acquired to determine the different positions that the measurement device may be driven to. That is, the measurement positions of the measurement device may be determined to correspond to the positions along the longitudinal axis of the guiding tube at which the tracking values had be determined.

By employing a system in which a (relative) measurement position for positioning a measurement device is determined based on a guiding tube reconstruction from tracking data, the necessity of attaching a tracking sensor to the measurement device may be avoided. This renders the current method agnostic with respect to the Invivo Dosimetry measurement technology used. Accordingly, it may be employed for any (ID) measurement device that fits a guiding tube which may be accurately reconstructed using tracking data.

Further, since the positions of the measurement device are determined by means of a reconstruction of the guiding tube, no tracking needs to be performed during the dose delivery process itself, thereby avoiding potential disturbances of the tracking data caused by the sources of radioactive radiation.

In some embodiments, the tracking data is acquired using electromagnetic and/or fiber-optic tracking.

In some embodiments, the tracking data may particularly be acquired using electromagnetic (EM) tracking. Even more particularly, the tracking data may be obtained using an EM tracked stylet that is introduced into the guiding tube to be reconstructed and has a respective EM tracking sensor attached thereto. A field generator may be provided for generating a magnetic field. The thus generated magnetic field induces a magnetic field in the tracking sensor of the EM tracked styled, thereby allowing a tracking of the sensor.

Alternatively or additionally, fiber-optic tracking may be used to acquire the tracking data. A fiber-optic tracking system particularly includes an optical fiber including a sensor and a light source. The sensor is adapted to modify the optical signals emanating from the light source in accordance with the position of the fiber in the guiding tube and to provide them to a respective detector, which derives the tracking data based on the modified optical signals.

Other tracking technologies such as, for example, radiography technologies, CT scan and/or magnetic resonance imaging may also be realized for one embodiment of the system.

In some embodiments, the system further comprises a computation unit for receiving measurement position data indicating the at least one measurement position relative to the at least one source position, and for deriving, based on a therapy plan, predicted dose data for the at least one measurement position.

The system may further comprise a computation unit that is configured to determine predicted dose data indicating the predicted dose, dose rate and/or dose distribution on the basis of a therapy plan. Hereby, the computation unit may particularly be configured to compute such predicted dose data for each of the measurement positions previously determined by the determination unit.

The computation unit is configured to receive, from a database, a treatment plan. This database may be comprised in a treatment planning system (TPS), a treatment control system or the like. In some embodiments, the computation unit particularly receives the dwell times of the source according to the treatment plan and the respective activity of the source.

The measurement position data may comprise one or more of the (relative) measurement positions at which the measurement device shall be positioned throughout the brachytherapy. The measurement position data may particularly comprise one particular (relative) measurement position, at which the measurement device is positioned to measure the dose delivered by its corresponding source. The computation unit may determine, from the measurement position data, the at least one relative measurement position for the measurement device and then calculate, using the dwell time and activity of the respective source to be measured at said measurement position, corresponding predicted dose data indicating the predicted dose and/or dose rate and/or dose distribution that is anticipated according to the therapy plan to be delivered (and, thus, measured by the measurement device) at said at least one measurement position. In this context, it shall be understood that the term predicted dose distribution may refer to a dose distribution at a single measurement position or may refer to a dose distribution at multiple measurement positions. It shall further be understood that the dose and/or dose rate and/or dose distribution particularly allow for drawing conclusions as to the amount of radiation that should be delivered to the target area in case the treatment occurs according to the therapy plan.

In accordance with these embodiments, it is possible to predict the dose (rate) that would be determined by the measurement device at each of the measurement positions. This may particularly allow a user to (manually) place the measurement device at a measurement position which, in accordance with the user's understanding, would exhibit a sufficiently accurate measurement of the radiation dose.

In some embodiments, the system further comprises a verification unit for deriving, based on radiation data acquired by the at least one measurement device at the at least one measurement position, delivered dose data of the dose delivered by the at least one source of radioactive radiation at the at least one measurement position, and comparing said delivered dose data to the predicted dose data derived for that particular measurement position.

In some embodiments, a verification unit is provided to verify that the dose that has been anticipated to be delivered to the target area upon placement of the source of radioactive radiation has actually been delivered and that no errors have occurred. This is achieved by comparing the calculated predicted dose data - which corresponds to what has been expected by the therapy plan - and the actually delivered dose data with one another and by determining their deviation from one another.

In order to perform such a comparison, the verification unit is configured to compute respective delivered dose data indicative of the dose and/or dose rate and/or dose distribution delivered to the target area. This delivered dose data may hereby particularly computed from radiation data that has been acquired by the measurement device at the determined measurement position. In that context, radiation data may particularly refer to the (local) radiation dose and/or dose rates (doses per time interval) the sensor of the respective measurement device has experienced at one or more measurement positions.

The verification unit is further configured to receive the predicted dose data from the computation unit indicating the dose that has been anticipated to be determined for the one or more measurement position according to the treatment plan. The verification unit is configured to compare the predicted dose data and the delivered dose data with one another. By means of the comparison between the delivered dose data indicating the delivered dose (rate) and the predicted dose data indicating the predicted dose (rate) that has been anticipated according to the therapy plan, the quality assurance of the process may be improved.

To that end, it may be determined whether the treatment plan and the corresponding brachytherapy treatment lead to the desired results or whether an adaption of the therapy plan may be necessary. More specifically, it may be determined from such a comparison, that the delivered and the predicted dose deviate from one another. Such a deviation may particularly be considered as an indication that the radiation emitted by the source is absorbed stronger or is not absorbed as well as planned and/or that the source is not properly placed and, thus, fails to irradiate the target area correctly. This would ultimately result in an adaption of the therapy plan.

When assessing the tolerance of the deviation between the delivered and predicted dose data, one should consider that measurement and calculation errors in deriving the delivered dose data may occur, i.e. that the delivered (measured) dose distribution will naturally deviate from the predicted dose distribution by a respective distribution delta. This deviation due to measurement and calculation errors may vary depending on the measurement device used to acquire the radiation data as well as the calculation approximations performed during calculation of the delivered dose data based on said radiation data. Accordingly, the distribution delta may be used as a threshold value for a respective therapy plan adaption and/or error detection. That is, if the distribution delta is inside a pre-defined tolerance the treatment continues without deviating from the therapy plan. If the distribution delta is not inside a pre-defined tolerance the treatment may continue with an adapted or re-scheduled therapy plan and/or may indicate an error.

The threshold value or distribution delta may particularly be configured to lie in the range between 1% and 20% deviation, even more particularly between 1% and 5% deviation. In some embodiments, when comparing the dose distribution, a common threshold for a deviation may even more particularly be configured to be a deviation in the range of less than 1%. In some embodiments, it may be determined, e.g. by a respective user configuration, that the measured and predicted dose shall not deviate at all.

The verification unit therefore allows an improved quality assurance of the brachytherapy treatment and, accordingly, enables a detection of inaccuracies in the treatment plan and a subsequent adjustment of the treatment plan, thereby improving the overall brachytherapy treatment provided.

In some embodiments, the system further comprises a display unit for generating a first graphical representation of the delivered dose data and a second graphical representation of the predicted dose data, and jointly displaying the first graphical representation and the second graphical representation.

In this context, the term display unit may refer to any kind of display that is configured to visualize the data indicated the delivered dose and the predicted dose for a user. The display unit may for example be a computer screen and/or an LCD display or the like. The display unit may be provided as part of the system or may be an external entity that is connectable to the system.

The term first and second graphical representation particularly refers to a visualization of the delivered dose data and the predicted dose data, respectively. The graphical representation may be two- or three-dimensional. In some embodiments, the delivered dose and the predicted dose may be plotted as a function of time and overlaid in a respective coordinate system. In these cases, the graphical representation corresponds to a graphical representation of the overlaid dose values in the coordinate system.

In some embodiments, the dose distribution of the delivered and predicted dose among different positions in the target area may be determined from the delivered and predicted dose data. In these cases, the graphical representation may particularly show a distribution map of the delivered and/or predicted dose data, either in an overlaid manner or next to one another. By overlaying the graphical representations or by displaying them side by side, the display unit jointly displays the first and second graphical representation.

It shall be understood that the display unit may be configured to display further graphical representations. In some particular embodiments, the display unit is also configured to display a graphical representation of the tracking data and/or a graphical representation of diagnostic image data obtained by a medical imaging means, such as X-ray scanning, computed tomography (CT), X-ray angiography, positron emission tomography (PET), single photon emission computed tomography (SPECT), ultrasound imaging, or the like. In some particular embodiments, the tracking data and the diagnostic image data are co-registered and jointly visualized by the display unit. Hereby, the term co-registration particularly refers to a procedure in which it is determined which image position in the tracking data corresponds to which image position of the diagnostic image data. It shall be understood that, the precise details on the co-registration depend on the tracking modality and/or the medical imaging modality used. The reconstruction of the one or more guiding tubes using tracking data will always result in a path in three-dimensional space which is associated with the tracking system. These tracking data may be co-registered to a corresponding diagnostic image from the diagnostic image data and may be jointly displayed by displaying the reconstructed guiding tube patch on top of said diagnostic image. Hereby, the diagnostic image may be derived from diagnostic image data that has previously been acquired for the patient. Alternatively, the diagnostic image data may be acquired while the tracking data is shown.

The first and second graphical representation as well as the graphical representation of the tracking data, the diagnostic image data and/or a combination thereof may particularly be displayed by the display unit as part of a respective user interface. This user interface may hereby comprise further elements, such as input elements allowing the user to control the system or elements for presenting further information to the user.

The co-registration of the diagnostic image data together with the tracking data may particularly be used in cases where the tracking data alone is not sufficient to provide accurate tracking results. In this case, an additional image processing step may be introduced, in which a rather narrow region of interest around the patch reconstructed from the tracking data is processed using the diagnostic image data. This might improve the accuracy of the tracking.

In some embodiments, the system further comprises an indication unit for triggering at least one indication if the delivered dose data and the predicted dose data deviate from one another by a pre-defined tolerance.

As indicated herein above, the verification unit may be able to detect a deviation between the delivered dose data and the predicted dose data. Such a deviation may allow to identify specific error scenarios. In some embodiments, such a deviation may be considered as an indication for a misplacement of the source of radioactive radiation and/or of the measurement device. More particularly, such a deviation may be indicative of the guiding tubes or channels having been switched, so that the source is currently guided by a wrong guiding tube, such as the guiding tube supposed to be used for the measurement device or an entirely different guiding tube). Likewise, a deviation may indicate that the measurement device may not be properly positioned.

In order to signal such an error to a user, an indication unit may be provided which is configured to trigger at least one indication. The indication may hereby correspond to an audible, visible or tactile alarm. This alarm may show that the distribution delta is not inside a pre-defined tolerance. The user may then decide how to proceed or if the treatment should be cancelled.

In some embodiments, the system further comprises an optimization unit for optimizing the at least one measurement position to an at least one optimized measurement position on the basis of the predicted dose data predicted based on the therapy plan. In some embodiments, the optimization unit is further provided for optimizing an insertion sequence of a plurality of guiding tubes on the basis of predicted dose distribution data predicted based on a therapy plan.

In some embodiments, position optimization of the measurement position may be performed based on the predicted dose distribution as derived from respective predicted dose distribution data. That is, a measurement position may be selected such as to be provided in a region in which the best measurement results may be obtained. Hereby, the optimization may rely on any numerical optimization method, but may particularly be performed using one or more of the following optimization methods:
Greedy optimization
Gradient-based optimization (e.g. Powel's search, conjugate gradient approaches, or more advanced (1-)BFGS approaches).

Stochastic optimization, (e.g. simulated annealing or genetic algorithms).

As indicated, the optimization unit may particularly be used to optimize the position of the measurement device inside the guiding tube. Further, the optimization unit may also be configured to optimize the insertion sequence of the respective guiding tubes. That is, the optimization method may use the predicted dose data and, optionally, further information derived from the therapy plan to determine an insertion sequence which will result in the best signal-to-noise ratio (SNR).

In that context, one particular approach of optimizing the position of the measurement device inside a particular guiding tube is the use of a penalty function in the predicted dose distribution. Hereby, all possible measurement positions in the guiding tubes used for the measurement devices (i.e. excluding the guiding tubes used for the sources) may be provided and then, iteratively, the guiding tubes as well as the measurement positions therein may be selected based on the dose distribution gradient in the penalty function which is supposed to optimize the position in accordance with the optimum signal-to-noise ratio that may be achieved for the predicted dose distribution.

By means of this optimization, the positioning of the ID measurement devices may be improved and, thus, the accuracy of the measurement performed using the measurement devices may be enhanced. This results in a better QA compared to approaches in which the position of the ID measurement devices is not optimized.

It shall be understood that, in the same manner, the insertion sequence for guiding tubes for the sources of radioactive radiation may be optimized. This may also be particularly achieved by a respective penalty function in the predicted dose distribution. More specifically, the predicted dose distribution gradient is penalized by the penalty function. Hereby, the guiding tubes to be placed, in particular needles to be placed, are iteratively selected based on the gradient in the penalty function. This mathematical approach hereby translates into selecting the tube that has the most impact on the dose distribution in the organs at risk.

In some embodiments, the optimizing of the at least one measurement position of the at least one measurement device to the at least one optimized measurement position comprises determining at least one further measurement position of at least one further measurement device, optimizing the at least one measurement position of the at least one measurement device to the at least one optimized measurement position depending on the at least one further measurement position of the at least one further measurement device; and/or optimizing the at least one further measurement position of the at least one further measurement device to at least one further optimized measurement position depending on the at least one measurement position of the at least one measurement device.

In some cases, it may be beneficial to use multiple measurement devices at various measurement positions inside the patient to measure the radiation dose that is delivered by one or more sources of radioactive radiation. If, in such a case, the above-mentioned optimization method is used, the optimized measurement positions for the multiple measurement devices may overlap.

In order to prevent placing multiple measurement devices at the same measurement position, the positioning of the measurement devices at their respective measurement positions may be performed based on a desired distance between the measurement devices. More particularly, when determining a first and a second measurement position for positioning a first and a second measurement device which shall measure the dose delivered by one or more sources of radioactive radiation, the determination may take into account that the first and second measurement position shall be distanced from one another by a predefined distance.

On possible approach to consider the desired distances in the optimizing of the measurement position is the employment of a penalty function to penalize certain distances between the measurement devices. More specifically, a penalty function may be added that is based on the desired distances between the individual (e.g. first and second) measurement devices, which penalized the distance between these measurement device. By penalizing the distances, an optimized measurement position may be found for each one of the individual measurement devices. Hereby, the optimized measurement positions for each of the measurement devices may particularly be determined by penalizing the distance between a set of candidate positions for one or more second measurement devices and an optimized position of a first measurement device and vice versa.

In some embodiments, the distances between the measurement positions of particularly individual measurement devices used for Invivo dosimetry may be penalized for a particular region of interest. In this case, a penalty function may be used that penalizes only the distance between the position of a first measurement device and a single second measurement device which is found to be the measurement device that is closest to the first measurement device. This may reduce the processing power necessary to determine the optimized measurement positions for multiple ID devices.

In some embodiments, the optimizing of the at least one measurement position to the at least one optimized measurement position further comprises identifying a plurality of candidate positions for the measurement device and predicting, based on the therapy plan, the predicted dose data for each of the plurality of candidate measurement positions. Further, the optimizing comprises determining, from the plurality of candidate measurement positions, at least one optimized measurement position by applying a penalty method to the predicted dose data. In some embodiments, applying the penalty method to the predicted dose data comprises applying a plurality of penalty functions and joining the plurality of penalty functions in an objective function using a weighted sum.

In some embodiments, the optimizing of the measurement position may be performed based on the predicted dose distribution by deriving one or more positions which may be potential candidates for placing the measurement devices and determining the dose distribution for each of these candidate positions. The (optimized) measurement position may then be selected from the candidate positions by applying a penalty method.

More specifically, the candidate positions are identified which are in or near the target area, in a regions of interest or organs at risk, which are not yet taken by the needles, implants or guiding tubes used for therapy delivery, and which are not in a no-go zone.

Using the tracks resulting from the plurality of candidate positions, the optimization unit then computes the predicted dose or dose rate for each candidate position along said track on the basis of the predicted dose data. Alternatively or additionally, the optimization unit may also obtain the predicted dose or dose rate from the computation unit, whereby the computation unit is configured to derive the predicted dose (rate) from the predicted dose data. Hereby, a numerical optimization employing a penalty method may particularly be used. Accordingly, the requirements for the measurement position at which the measurement device shall be placed are translated in a set of representative penalty functions. These penalty functions can be used in a (weighted) sum to formulate the objective function that is to be optimized by the optimization software.

In this context, the following penalty functions may particularly be used for optimizing the measurement position of at least one ID measurement device:
Positioning ID measurement devices in areas with high spatial gradients in the predicted dose distribution are penalized by computing the spatial gradient amplitude in the final treatment plan at each candidate position. If a higher penalization is required, one might opt for using the squared gradient magnitude, or consider the sum or maximum of the x, y, and z components of the spatial gradient in the final treatment plan.

Positioning measurement devices in areas with low dose or dose rate in the predicted dose distribution are penalized by computing the difference between a minimal acceptable level and the predicted level of the dose or dose rate. Alternate formulations may substitute this difference in a (single-sided) quadratic or higher order polynomial, in an exponential function, or in a Heavyside function.

Measurement positions close to regions of interest are prioritized by computing the distance between a candidate position to such a region of interest. Alternate formulations may substitute this distance in a (single-sided) quadratic or higher order polynomial, in an exponential function, or in a Heavyside function.

In addition, variations on this penalty function may be formulated by computing the distance to local minima/maxima in the planned dose distribution to propose measurement positions such that the dose or dose rate is measured where it matters most.

The penalty functions may be joined in an objective function using a weighted sum. The weightings may be assigned (automatically and/or by the user) to alter the balance between the individual aspects in the problem.

Subsequently, a core optimization method may minimize the complete objective function by moving the measurement devices through the solution space. This core method can rely on any numerical optimization method, but may particularly be performed by greedy optimization, gradient-based optimization and/or stochastic optimization.

In support of being able to identify errors scenarios, another penalty function may be introduced, computing for example the correlation of the predicted dose or dose rate signal that is anticipated according to the plan with the dose or dose rate signal in case the guiding tubes are connected wrongly. This will drive the solution towards a position at which the measurement result changes most if an error occurs. Again, alternate formulations may substitute the correlation in a (single-sided) quadratic or higher order polynomial, in an exponential function, or in a Heavyside function. In addition, instead of using correlation measures, on might rely on simple difference measures or more advanced mutual information measures.

In some embodiments, the optimizing of the measurement position for the measurement device(s) may also be based on the sequence in which the one or more individual sources are positioned. This is particularly so since a location in the target area at which a low spatial gradient occurs according to the therapy plan may exhibit a higher spatial gradients during dose delivery. In this case, it may be beneficial to compute the penalty functions for the measurement devices over all time-points during the delivery in order to track changes in the spatial gradient during this process.

In some embodiments, the optimization unit is further provided for receiving at least one change indication of a change in the therapy plan, and adjusting the optimizing of the at least one measurement position to the at least one optimized measurement position based on the at least one change indication.

As indicated herein above, brachytherapy treatments may last over an extended period of time. During this time, the changes in the cancerous growths and/or tumors may necessitate an adjustment of the therapy plan. This adjustment may particularly be performed manually by a user through the user interface and/or respective user input means. In case the therapy plan is changed, the optimization unit may receive a respective indication of the change comprising, among others, changes in the planned source positions, in the planned dwell time of the sources, the sources used and the like. Based on this indication, the optimization unit may adjust the optimizing of the measurement position accordingly. This may particularly be performed by re-calculating the predicted dose data comprising the predicted dose and/or dose rate for the target area and using the adjusted predicted dose data in order to derive the optimized measurement position from the plurality of candidate positions.

In a further aspect, a computer program for controlling the above-described system is provided, which, when executed by a processing unit, is adapted to perform the method steps according to the invention. In an even further aspect, a computer-readable medium is provided having stored thereon the above-cited computer program.

It shall be understood that the system for brachytherapy treatment may be implemented by means of a processing unit. Hereby, the input unit, the reconstruction unit, the determination unit, the computation unit drive unit, the verification unit and the optimization unit may be implemented as modules in the processing unit. The functionality of these modules may in particular be implemented by means of a respective algorithm. This algorithm may in particular be implemented using a machine learning algorithm implemented on said processing unit including said modules.

It shall be understood that the system of claim 1, the method of claim 13, the computer program of claim 14, and the computer-readable medium of claim 15 have similar and/or identical preferred embodiments, in particular, as defined in the dependent claims.

It shall be understood that a preferred embodiment of the present invention can also be any combination of the dependent claims or above embodiments with the respective independent claim.

These and other aspects of the invention will be apparent from and elucidated with reference to the embodiments described hereinafter.

### BRIEF DESCRIPTION OF THE DRAWINGS

In the following drawings:
Fig. 1 schematically illustrates a system for determining a measurement position of an invivo dosimetry (ID) measurement device used for quality assurance in brachytherapy treatment according to an embodiment.
Fig. 2 schematically illustrates a method for determining a measurement position of an ID measurement device used for quality assurance in brachytherapy treatment according to an embodiment.
Fig. 3 schematically illustrates a system for determining and optimizing a measurement position of an ID measurement device according to a further embodiment.
Fig. 4 illustrates a method for optimizing a measurement position of an ID measurement device
Fig. 5 diagrammatically illustrates a system for brachytherapy according to embodiments of the invention.

### DETAILED DESCRIPTION OF EMBODIMENTS

The illustration in the drawings is schematically. In different drawings similar or identical elements are provided with the same reference numerals.

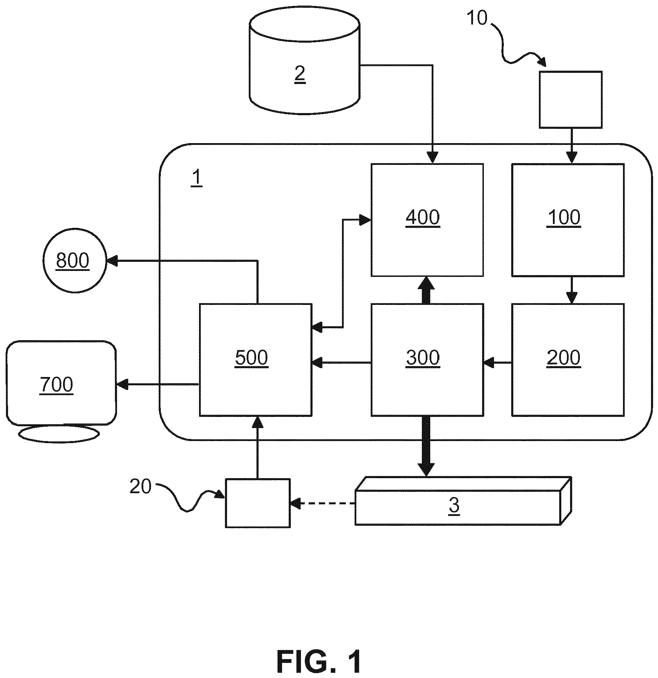

Fig. 1 schematically represents a system 1 for supporting the positioning of invivo dosimetry (ID) measurement devices inside a guiding tube, such as a catheter, according to an exemplary embodiment. The system 1 comprises an input unit 100, a reconstruction unit 200, a determination unit 300, a computation unit 400, a verification unit 500, a display unit 700 and an indication unit 800. The system 1 is further connected to a database 2, which in the exemplary embodiment of Fig. 1 is configured as a therapy planning system, and to a control unit 3 for controlling the positioning of one or more ID devices configured to acquire radiation data 20.

The input unit 100 receives tracking data 10 of at least one measurement guiding tube. In the exemplary embodiment according to Fig. 1, the tracking data 10 corresponds to electromagnetic tracking data 10 that has been obtained by inserting an electromagnetic tracking element into at least one guiding tube and by tracking the tracking element at a plurality of positions along the longitudinal length of said guiding tube. Electromagnetic tracking of a guiding tube using an electromagnetic tracking element is described in more detail in WO2014013418.

The electromagnetic tracking data 10 is then provided to reconstruction unit 200. The reconstruction unit 200 uses the electromagnetic tracking data 10 acquired at each of a plurality of positions along the at least one guiding tube to reconstruct said measurement guiding tube. The reconstruction of the at least one measurement guiding tube is then provided from the reconstruction unit 200 to determination unit 300.

The determination unit 300 uses the reconstruction to determine a measurement position of the ID measurement device guided by the measurement guiding tube represented in that reconstruction. The measurement position is hereby determined relative to a corresponding source position of a respective source of radioactive radiation. In other words, the positional relationship between the measurement guiding tube and its corresponding source guiding tube, respectively, is determined, which, in turn, allows to determine the relative position of the ID measurement device relative to the position of the source which radiation shall be measured using said ID measurement device.

In the exemplary embodiment according to Fig. 1, this is achieved by using the same tracking system and same tracking element to track and reconstruct both, the at least one measurement guiding tube and the corresponding at least one source guiding tube. Using the same tracking system for both, the measurement guiding tube and the source guiding tube, intrinsically defines the reconstruction of both guiding tubes in the same coordinate system. This allows to directly derive the positional relationship from the acquired tracking data.

Based on this, the determination unit 300 obtains measurement position data indicating the measurement position of the ID measurement device relative to the corresponding source position of its respective source and provides this measurement position data to computation unit 400.

The computation unit 400 receives the measurement position data and derives the measurement position therefrom. Further, the computation unit 400 receives a therapy plan from the therapy planning system 2. In the exemplary embodiment according to Fig. 1, the computation unit 400 particularly receives the dwell time of the source indicated by the therapy plan along with information about the activity of the source. Using the dwell time and the activity of the source, the computation unit 400 then computes respective predicted dose data indicating the dose that is anticipated to occur at the measurement position. The computation unit 400 provides the predicted dose data to verification unit 500.

The determination unit 300 further provides the measurement position data indicating the at least one measurement position to control unit 3. Control unit 3 may be configured to automatically position the ID measurement device at the measurement position indicated in the measurement position data. Alternatively or additionally, the control unit may be configured to present the measurement position data to a user. The user may then be prompted to drive the ID measurement device to the measurement position manually.

The ID measurement device is then used to acquire radiation data 20 at the measurement position. The acquired radiation data 20 is provided to the verification unit 500. The verification unit 500 uses the radiation data 20 acquired at the measurement position to derive delivered dose data indicating the dose that has actually been delivered to the target area. The verification unit 500 then compares the delivered dose data with the predicted dose data received from the computation unit 400 in order to verify that the dose delivery has been performed as planned and in order to perform the requested quality assurance of the process.

In the exemplary embodiment according to Fig. 1, the verification unit then provides the delivered dose data and the predicted dose data to display unit 700. Display unit 700 generates a first graphical representation of the delivered dose data and a second graphical representation of the predicted dose data and jointly displays them on a display, such as a computer screen, to visualize them for a user. Hereby, the first graphical representation and the second graphical representation are particularly provided in the same coordinate system and, even more specifically, overlaid in order to allow for an easier comparison.

The system 1 is further provided with an indication unit 800. The indication unit 800 is communicatively connected to the verification unit 500 to output an indication of the verification result, i.e. of the result of the comparison between the delivered dose data and the predicted dose data. As an example, in case the verification unit 500 indicates that the comparison between the delivered dose data and the predicted dose data are both within predefined parameters, the indication unit 800 may indicate that the radiation delivery has been performed according to the plan, e.g. visually, by displaying a green light and/or audibly, by providing a sound, and/or in a tactile manner.

In some embodiments, the verification unit 500 may determine that the delivered dose data and the predicted dose data deviate from one another in a manner that exceeds a predetermined deviation threshold, i.e. that the dose actually delivered to the target area is significantly different from the predicted dose as anticipated according to the therapy plan. This may for example be a result of a misplacement of the source, such as an insertion of the source into an incorrect guiding tube. In this case, the indication unit 800 may raise an alarm, indicating that an error has occurred. This alarm may be a visual alarm, such as a red light, an auditory alarm and/or a tactile alarm.

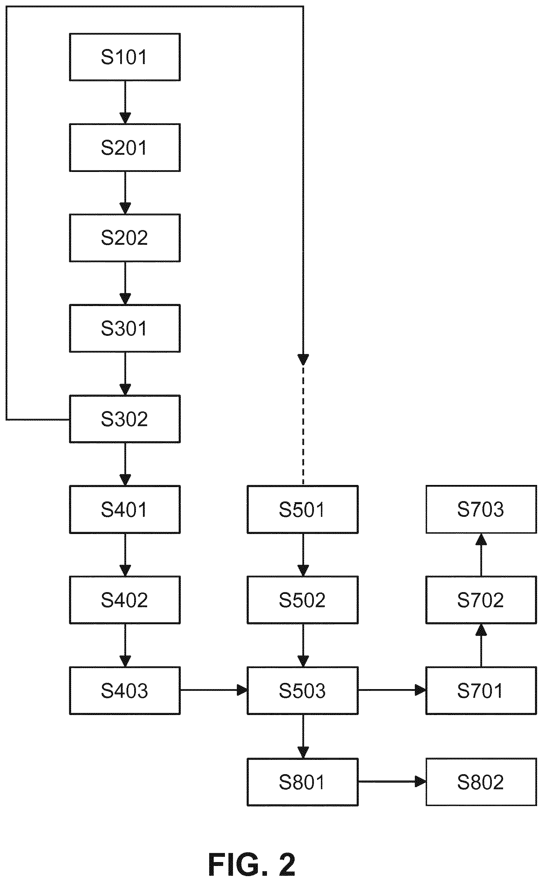

Fig. 2 schematically represents a method for determining a measurement position for an ID measurement device according to an embodiment. At step S101, the input unit 100 receives the tracking data 10 obtained for the measurement guiding tube to guide the respective ID measurement device. In step S201, the reconstruction unit 200 receives the tracking data 10 from input unit 100 and uses the tracking data 10 acquired to reconstruct the measurement guiding tube at step S202.

At step S301, the determination unit 300 receives the reconstruction of the measurement guiding tube and uses this reconstruction to determine a measurement position of the ID measurement device and to obtain respective measurement position data in step S302 which is subsequently sent to computation unit 400 and to control unit 3.

The measurement position data is received at computation unit 400 in step S401. In step S402, the computation unit 400 further receives a therapy plan from therapy planning system 2, in particular the dwell time and activity of the sources. Using the therapy plan, the computation unit 400 then computes, in step S403, predicted dose data of the anticipated dose at the measurement position and provides the predicted dose data to verification unit 500.

As indicated, control unit 3 also receives the measurement position data and prompts the ID measurement device to acquire radiation data 20 at the measurement position. The acquired radiation data 20 is provided to verification unit 500 in step S501. In step S502, the verification unit 500 uses the radiation data 20 to derive the delivered dose data at the measurement position indicating the dose actually delivered to the target area. In step S503, the verification unit 500 further receives the predicted dose data and compares the delivered dose data with the predicted dose data. Upon comparison, the verification unit 500 provides the delivered dose data and the predicted dose data to display unit 700.

The delivered dose data and the predicted dose data are received at display unit 700 in step S701. The display unit 700 generates, in step S702, a first graphical representation of the delivered dose data and a second graphical representation of the predicted dose data. In step S703, the display unit 700 then jointly displays the graphical representations to a user.

Further, the verification unit 500 may provide verification information regarding the comparison of the delivered dose data and the predicted dose data to indication unit 800. This verification information is received at indication unit 800 in step S801. In response to this, indication unit may, in step S802, output a respective indication, why may either be positive, i.e. showing that everything is going as planned, or negative, i.e. an alarm raised due to an error having occurred.

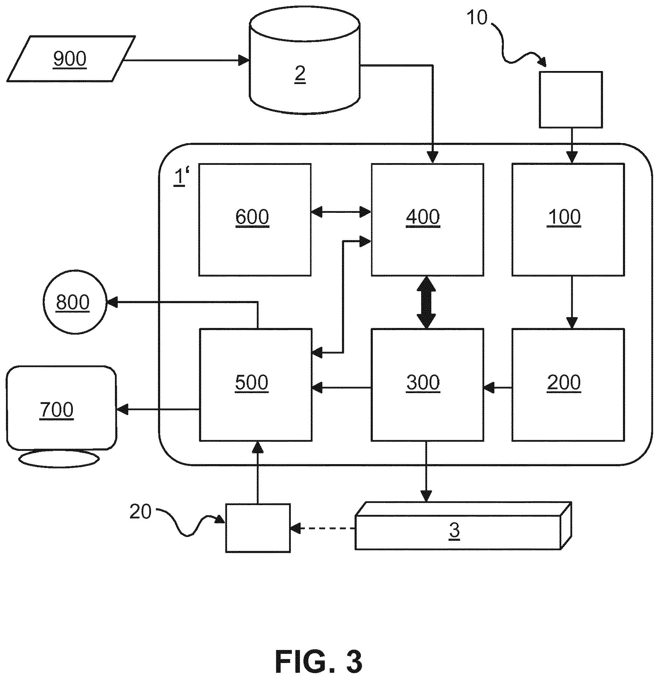

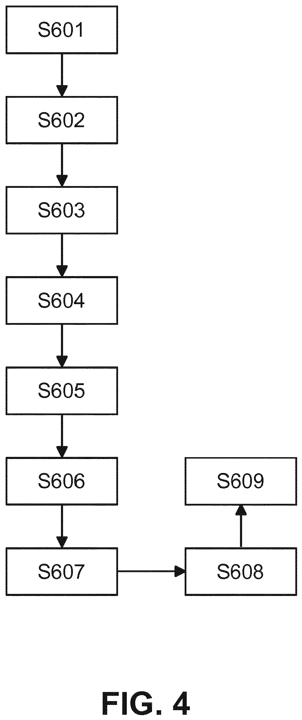

Fig. 3 schematically represents a modification of the system for supporting the positioning of ID measurement devices inside a catheter as represented in Fig. 1. The modified system 1' comprises an input unit 100, a reconstruction unit 200, a determination unit 300, a computation unit 400, a verification unit 500, a display unit 700 and an indication unit 800 which are configured in a similar manner as described in relation to Fig. 1. The system 1' is therefore also connected to a database, or therapy planning system, 2 and a control unit 3. Furthermore, the system 1' comprises an optimization unit 600 configured for optimizing the measurement position of the ID measurement devices as particularly described in relation to Fig. 4.

The procedures in the modified system 1' are performed in the same manner as described in relation to Fig. 1: The input unit 100 receives tracking data 10 of at least one measurement guiding tube and the reconstruction unit 200 uses the tracking data 10 to reconstruct said measurement guiding tube. The reconstruction of the measurement guiding tube is then provided to determination unit 300.

The determination unit 300 then uses the reconstruction to determine a measurement position of the ID measurement device guided by the measurement guiding tube as reconstructed. This is particularly achieved by the determination unit 300 deriving measurement position data indicating a plurality of positions along the measurement guiding tube, which may correspond to the positions at which the tracking data has been obtained, respectively. The determination unit 300 may then provide the measurement position data indicating the plurality of measurement positions to the computation unit 400.

The computation unit 400 receives the measurement position data and derives the plurality of measurement positions. Further, the computation unit 400 receives the therapy plan received from the therapy planning system 2. The computation unit 400 may then provide the plurality of measurement positions indicated in the measurement position data and the therapy plan to optimization unit 600.

The optimization unit 600 receives the measurement position data and the therapy plan and identifies a plurality of candidate positions from said measurement position data. These candidate positions hereby correspond to positions that are considered potential candidates for the optimized measurement position at which the ID measurement device shall be positioned. It shall be understood that the term plurality of candidate positions in this context may define all measurement positions indicated in the measurement position data or may refer to a subset of the plurality of measurement positions.

The optimization unit 600 then derives predicted dose data comprising, for each of the candidate positions, the predicted dose and/or dose rate anticipated at the plurality of candidate positions. The optimization unit 600 then applies a penalty method on the predicted dose data indicating the predicted dose (rate) at the plurality of candidate positions as described in more detail in relation to Fig. 4. Based on this penalty method, the optimized measurement position, i.e. the position at which the ID measurement device will provide the best measurement results is identified. The optimization unit 600 may then either adjust the measurement position data received to include the optimized measurement position or may generate optimized measurement position data indicating the optimized measurement position only. The optimization unit 600 may the provide the (optimized) measurement position data along with the predicted dose data indicating the predicted dose (rate) at the optimized measurement position to computation unit 400.

The computation unit 400 then provides the predicted dose data to verification unit 500 and the (optimized) measurement position data to the determination unit 300. The determination unit 300 then provides the (optimized) measurement position data indicating at least the optimized measurement position to control unit 3. Control unit 3 may then automatically position the ID measurement device at the optimized measurement position or, alternatively or additionally, display the optimized measurement position to a user to prompt said user to position the ID measurement device at the optimized measurement position.

The ID measurement device is then used to acquire radiation data 20 at the optimized measurement position. The radiation data 20 is provided to verification unit 500 which uses the radiation data 20 to derive delivered dose data indicating the actually delivered dose. The verification unit 500 then compares the delivered dose data and the predicted dose data as described herein above in relation to Fig. 1 and provides the verification information indicating the result of this comparison to display unit 700 and/or indication unit 800.

In the exemplary embodiment according to Fig. 3, the therapy planning system 2 comprises a user interface 900. The user interface 900 may be configured to allow a user to input changes to the therapy plan into the therapy planning system 2. In this case, the therapy planning system 2 provides a respective change indication to the system 1' and, thereby, to optimization unit 600. The change indication comprises and information about a change of the therapy plan, such as a change of the source position, the source activity, the dwelling time or the like. The optimization unit 600 is then configured to adapt the optimization in accordance with the change indication. That is, the determination of the predicted dose data is adjusted in accordance with the new therapy plan and the optimization procedure is performed in accordance with the adjusted predicted dose data.

Fig. 4 schematically represents a method for optimizing the measurement position as may particularly be performed by the optimization unit 600 as part of the system 1'. In step S601, the measurement position data and the therapy plan are received at the optimization unit 600. In step S602, the optimization unit 600 identifies, based on the measurement position data, a plurality of candidate positions. In step S603, the optimization unit 600 determines, based on the therapy plan, predicted dose data indicating the predicted dose (rate) at each of the candidate positions - and, thus, the predicted dose distribution among the candidate positions, and uses the predicted dose data to optimize the measurement position by applying a penalty method. In the exemplary embodiment according to Fig. 4, the optimization is performed as a numerical optimization which exhibits the best QA by translating the following considerations into respective penalty functions:
Positioning of ID measurement devices should be avoided in areas with high spatial gradients in the planned radiation dose distribution. Otherwise, small errors in relative positioning result in large deviations for the measured radiation dose.

The ID measurement devices may not be sensitive enough to measure the radiation dose or radiation dose rate that is delivered from all source positions. Hence, areas, which exhibit low radiation dose according to the therapy plan should be avoided.

High confidence QA in crucial areas close to or inside target organs or organs at risk should be prioritized. Hence, placement of sensors or ID devices - if allowed - should be prioritized for these areas.

The sequence of source positions affects the evolution of the spatial dose gradient over time.

In step S604, the positioning of the ID measurement device in areas with high spatial gradients indicated by the predicted dose data is penalized by computing the spatial gradient amplitude in the final plan at each candidate position. If a higher penalization is required, one might opt for using the squared gradient magnitude, or consider the sum or maximum of the x, y, and z components of the spatial gradient in the final plan.

In step S605, the positioning of the ID measurement device in areas with low dose or dose rate as indicated in the predicted dose data is penalized by computing the difference between a minimal acceptable level and the predicted level of the dose or dose rate. Alternate formulations may substitute this difference in a (single-sided) quadratic or higher order polynomial, in an exponential function, or in a Heavyside function.

In step S606, any candidate position for the measurement position that is close to or inside target organs or organs at risk, i.e. close to particularly selected regions of interest, is prioritized by computing the distance between the respective candidate position to such a region of interest. Alternate formulations may substitute this distance in a (single-sided) quadratic or higher order polynomial, in an exponential function, or in a Heavyside function. In addition, variations on this penalty function may be formulated by computing the distance to local minima/maxima in the planned dose distribution to propose measurement positions such that the dose or dose rate is measured where it matters most.

In step S607, the sequence in which the sources of radioactive radiation are placed is considered by computing over all time points during the delivery.

Further, in step S608, a penalty function is introduced to identify delivery failure situations, such as exchanging of source and/or measurement guiding tubes or the like. This penalty function may particularly compute a correlation of the predicted dose (rate) and the dose rate in case of such an exchange of the source and/or measurement guiding tubes. This may drive the solution towards an optimized measurement position at which the measurement performed by the ID measurement device would change most in case of such a failure occurrence. In this case also, alternate formulations may be used to substitute the correlation in a (single-sided) quadratic or higher order polynomial, in an exponential function or a Heavyside function. Alternatively or additionally, a difference measure may be used to identify delivery failures.

In step S609, the different penalty functions are joined in an objective function using a weighted sum. The weightings may be assigned (automatically and/or by the user) to alter the balance between the individual aspects in the problem. This allows to adjust the optimization in accordance with the main objective to be achieved by it (improved quality assurance, fast delivery failure detection, etcetera).

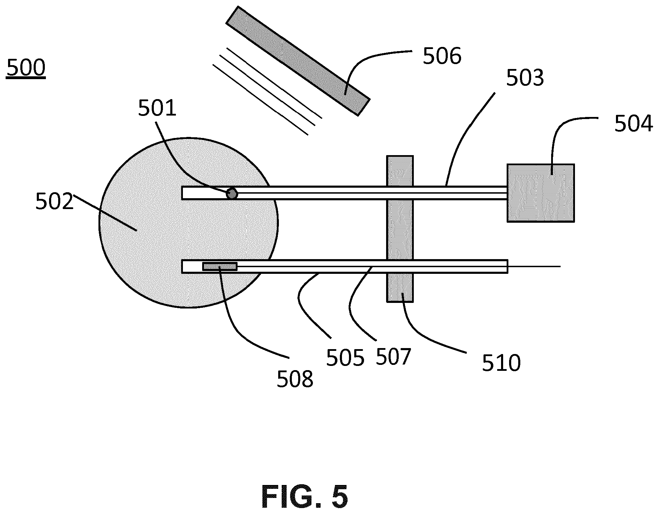

Fig. 5 diagrammatically illustrates a system for brachytherapy 500 according to embodiments of the invention. Guiding tubes 503, 504 may be inserted into a treatment target 502, which may be a prostate. In order to more accurately position the guide tubes, a grid 510 may be used. An afterloader 504 can be used to move the source of radioactive radiation 501 into the guiding tube 503. Fig 5. further shows a field generator 506 that is configured for generating an EM field that in turn can be used for tracking the position of the guiding tubes 503 and 504 moving through this EM field as explained above. In this way the position of the measurement device 508 relative to the source of radioactive radiation 501 can be obtained.

Although the above-described embodiments relate to high dose rate (HDR) brachytherapy, it shall be understood that the invention is not limited to HDR brachytherapy, but may also be applied to low dose rate (LDR) brachytherapy or pulsed dose rate (PDR) brachytherapy.

Further, it shall be understood that, although in the above-described embodiments, the system is implemented as part of a therapy planning system (TPS) for brachytherapy planning, the system may likewise be implemented in a therapy control system (TCS), a therapy verification system (TVS) or may be implemented as part of an afterloader for driving the sources of radioactive radiation through their respective guide wires.

Although in the above-mentioned embodiments, the optimization unit is configured to optimize the measurement positions for the individual ID measurement devices, it shall be understood that the optimization unit may likewise be employed to optimize source positioning.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

Procedures like the reconstruction of the guiding tubes, the determination of the measurement positions, the optimization of the measurement positions, the deriving of the predicted or delivered dose data, the comparing of the predicted and delivered dose data, the manual and/or automatic controlling, the indicating by an audible and/or tactile and/or visible alarm, the visualization, et cetera performed by one or several units or devices can be performed by any other number of units or devices. These procedures in accordance with the invention can hereby be implemented as program code means of a computer program and/or as dedicated hardware.

A computer program may be stored and/or distributed on a suitable medium, such as an optical storage medium or a solid-state medium, supplied together with or as part of other hardware, but may also be distributed in other forms, such as via the Internet or other wired or wireless telecommunication systems.

Any reference signs in the claims should not be construed as limiting the scope.

The invention relates to a system for brachytherapy treatment comprising an input unit for receiving tracking data comprising a plurality of tracking values acquired at a plurality of positions along at least one guiding tube, a reconstruction unit for obtaining a reconstruction of said at least one guiding tube based on the tracking data and a determination unit for determining, based on the reconstruction, at least one measurement position of at least one measurement device guided by said at least one guiding tube relative to at least one source position of at least one source of radioactive radiation.

By means of such a system the positioning of the invivo dosimetry measurement devices may be performed with higher accuracy, and, thereby, an improvement of the quality assurance procedure in brachytherapy is achieved.

## Claims

1. A system for brachytherapy treatment comprising at least one guiding tube for guiding sources of radioactive radiation into a patient;
at least one measurement device guided by said at least one guiding tube relative to at least one source position of at least one source of radioactive radiation;
an input unit (100) adapted to receive tracking data comprising a plurality of tracking values acquired at a plurality of positions along said at least one guiding tube;
a reconstruction unit (200) adapted to obtain a reconstruction of said at least one guiding tube based on the tracking data;
the system being **characterised by** further comprising:
a determination unit (300) adapted to determine, based on the reconstruction, at least one measurement position of the at least one measurement device.

2. The system according to claim 1, wherein the tracking data is acquired using electromagnetic and/or fiber-optic tracking.

3. The system according to claim 1, further comprising
a computation unit adapted to receive measurement position data indicating the at least one measurement position relative to the at least one source position, and
to derive, based on a therapy plan, predicted dose data for the at least one measurement position.

4. The system according to claim 3, further comprising
a verification unit adapted to derive, based on radiation data acquired by the at least one measurement device at the at least one measurement position, delivered dose data of the dose delivered by the at least one source of radioactive radiation at the at least one measurement position, and
to compare said delivered dose data to the predicted dose data derived for that particular measurement position.

5. The system according to claim 4, further comprising
a display unit adapted to generate a first graphical representation of the delivered dose data and a second graphical representation of the predicted dose data, and
to jointly display the first graphical representation and the second graphical representation.

6. The system according to claim 4, further comprising
an indication unit adapted to trigger at least one indication if the delivered dose data and the predicted dose data deviate from one another by a pre-defined tolerance.

7. The system according to claim 3, further comprising
an optimization unit adapted to optimize the at least one measurement position to an at least one optimized measurement position on the basis of the predicted dose data predicted based on the therapy plan.

8. The system according to claim 7, wherein
the optimization unit is further adapted to optimize an insertion sequence of a plurality of guiding tubes on the basis of predicted dose distribution data predicted based on a therapy plan.

9. The system according to claim 7, the optimization unit is further adapted to perform the optimizing of the at least one measurement position of the at least one measurement device to the at least one optimized measurement position comprising the following steps:
determining at least one further measurement position of at least one further measurement device;
optimizing the at least one measurement position of the at least one measurement device to the at least one optimized measurement position depending on the at least one further measurement position of the at least one further measurement device; and/or
optimizing the at least one further measurement position of the at least one further measurement device to at least one further optimized measurement position depending on the at least one measurement position of the at least one measurement device.

10. The system according to claim 7, wherein the optimization unit is further adapted to perform the optimizing of the at least one measurement position to the at least one optimized measurement position comprising the following steps:
identifying a plurality of candidate positions for the measurement device;
predicting, based on the therapy plan, the predicted dose data for each of the plurality of candidate measurement positions; and
determining, from the plurality of candidate measurement positions, at least one optimized measurement position by applying a penalty method to the predicted dose data.

11. The system according to claim 10, wherein the applying the penalty method to the predicted dose data comprises applying a plurality of penalty functions and joining the plurality of penalty functions in an objective function using a weighted sum.

12. The system according to claim 7, wherein the optimization unit is further adapted to receive at least one change indication of a change in the therapy plan; and to adjust the optimizing of the at least one measurement position to the at least one optimized measurement position based on the at least one change indication

13. A method for quality assurance during brachytherapy treatment, the method comprising the steps of:
receiving tracking data comprising a plurality of tracking values acquired at a plurality of positions along at least one guiding tube;
obtaining a reconstruction of said at least one guiding tube based on the tracking data; and
determining, based on the reconstruction, at least one measurement position of at least one measurement device guided by said at least one guiding tube relative to at least one source position of at least one source of radioactive radiation.

14. A computer program which, when executed by a processing unit, is adapted to perform a method comprising the steps of:
receiving tracking data comprising a plurality of tracking values acquired at a plurality of positions along at least one guiding tube;
obtaining a reconstruction of said at least one guiding tube based on the tracking data; and
determining, based on the reconstruction, at least one measurement position of at least one measurement device guided by said at least one guiding tube relative to at least one source position of at least one source of radioactive radiation.

15. A computer program according to claim 14 further configured for controlling a system according to anyone of claim 1-12.

## Patentansprüche

1. Ein System für die Brachytherapie-Behandlung mit mindestens einer Führungsröhre zum Leiten von radioaktiven Strahlungsquellen in einen Patienten; mindestens ein Messgerät, das von der mindestens einen Führungsröhre relativ zu mindestens einer Quellenposition mindestens einer radioaktiven Strahlungsquelle geleitet wird;
eine Eingabeeinheit (100), die Nachverfolgungsdaten empfängt,
die mehrere Nachverfolgungswerte umfassen,
die an mehreren Positionen entlang der mindestens einen Führungsröhre erfasst wurden;
eine Rekonstruktionseinheit (200),
die beruhend auf den Nachverfolgungsdaten eine Rekonstruktion der mindestens
einen Führungsröhre abruft;
wobei sich das System dadurch auszeichnet, dass es zudem Folgendes umfasst:
eine Erkennungseinheit (300),
die beruhend auf der Rekonstruktion mindestens eine Messposition des mindestens
einen Messgeräts erkennt.

2. Das System gemäß Anspruch 1, wobei die Nachverfolgungsdat n mithilfe einer elektromagnetischen und/oder Glasfaser-Nachverfolgung erfasst werden.

3. Das System gemäß Anspruch 1, wobei
das System zudem eine Berechnungseinheit umfasst,
die Messpositionsdaten empfängt, die die mindestens eine Messposition relativ zur mindestens einen Quellenposition angeben, und
die beruhend auf einem Therapieplan die prognostizierten Dosisdaten für die mindestens eine Messposition ableitet.

4. Das System gemäß Anspruch 3,
das zudem eine Überprüfungseinheit umfasst,
die anhand der vom mindestens einen Messgerät an der mindestens einen Messposition erfassten Strahlungsdaten Daten über die von der mindestens einen radioaktiven Strahlungsquelle an der mindestens einen Messposition verabreichte Dosis ableitet und
die Daten über die verabreichte Dosis mit den für diese Messposition abgeleiteten prognostizierten Dosisdaten vergleicht.

5. Das System gemäß Anspruch 4, wobei dieses zudem Folgendes umfasst:
eine Anzeigeeinheit,
die eine erste grafische Darstellung der Daten über die verabreichte Dosis sowie eine zweite grafische Darstellung der Daten über die prognostizierte Dosis generiert, und
die die erste und zweite grafische Darstellung gemeinsam anzeigt.

6. Das System gemäß Anspruch 4, wobei dieses zudem Folgendes umfasst:
eine Hinweiseinheit, die mindestens einen Hinwies ausgibt, wenn die Daten über die verabreichte Dosis und die Daten über die prognostizierte Dosis um eine vordefinierte Toleranz voneinander abweichen.

7. Das System gemäß Anspruch 3, wobei dieses zudem Folgendes umfasst:
eine Optimierungseinheit, die die mindestens eine Messposition anhand
der im Rahmen des Therapieplans prognostizierten Dosisdaten auf mindestens eine optimierte Messposition optimiert.

8. Das System gemäß Anspruch 7, wobei die Optimierungseinheit zudem anhand der im Rahmen eines Therapieplans prognostizierten Dosisverteilungsdaten die Einführungsabfolge für die Führungsröhren optimiert.

9. Das System gemäß Anspruch 7, wobei die Optimierungseinheit zudem im Rahmen der folgenden Schritte das Optimieren
der mindestens einen Messposition des mindestens einen Messgeräts auf die mindestens eine optimierte Messposition durchführt:
Ermitteln mindestens einer weiteren Messposition des mindestens einen weiteren Messgeräts;
Optimieren der mindestens einen Messposition des mindestens einen Messgeräts auf die mindestens eine optimierte Messposition abhängig von der mindestens einen weiteren Messposition des mindestens einen weiteren Messgeräts; und/oder
Optimieren der mindestens einen weiteren Messposition des mindestens einen weiteren Messgeräts auf mindestens eine weitere optimierte Messposition abhängig von der mindestens einen Messposition des mindestens einen Messgeräts.

10. Das System gemäß Anspruch 7, wobei die Optimierungseinheit zudem im Rahmen der folgenden Schritte das Optimieren der mindestens einen Messposition auf die mindestens eine optimierte Messposition durchführt:
Ermitteln mehrerer möglicher Positionen für das Messgerät;
beruhend auf dem Therapieplan Prognostizieren der prognostizierten Dosisdaten für die einzelnen möglichen Messpositionen; und
Ermitteln unter den mehreren möglichen Messpositionen mindestens einer optimierten Messposition, indem auf die prognostizierten Dosisdaten eine Handicap-Methode angewandt wird.

11. Das System gemäß Anspruch 10, wobei das Anwenden der Handycap-Methode auf die prognostizierten Dosisdaten das Anwenden mehrerer Handycap-Funktionen sowie das Zusammenführen der Handycap-Funktionen in einer Zielfunktion mithilfe einer gewichteten Summe umfasst.

12. Das System gemäß Anspruch 7, wobei
die Optimierungseinheit zudem mindestens einen Änderungshinweis über eine Änderung des Therapieplans abruft; und
wobei
sie die Optimierung der mindestens einen Messposition auf die mindestens eine optimierte Messposition beruhend auf dem mindestens einen Änderungshinweis anpasst.

13. Eine Qualitätssicherungsmethode für eine Brachytherapie-Behandlung, wobei die Methode folgende Schritte umfasst:
Abrufen von Nachverfolgungsdaten, die mehrere Nachverfolgungswerte umfassen, die an mehreren Positionen entlang der mindestens einen Führungsröhre erfasst wurden;
beruhend auf den Nachverfolgungsdaten Abrufen einer Rekonstruktion der mindestens einen Führungsröhre;
beruhend auf der Rekonstruktion Ermitteln mindestens einer Messposition des mindestens einen Messgeräts, das von der mindestens einen Führungsröhre relativ zu mindestens einer Quellenposition mindestens einer radioaktiven Strahlungsquelle geleitet wird.

14. Ein Computerprogramm, das beim Ausführen durch eine Verarbeitungseinheit eine Methode mit den folgenden Schritten durchführt:
Abrufen von Nachverfolgungsdaten, die mehrere Nachverfolgungswerte umfassen, die an mehreren Positionen entlang der mindestens einen Führungsröhre erfasst wurden;
beruhend auf den Nachverfolgungsdaten Abrufen einer Rekonstruktion der mindestens einen Führungsröhre;
beruhend auf der Rekonstruktion Ermitteln mindestens einer Messposition des mindestens einen Messgeräts, das von der mindestens einen Führungsröhre relativ zu mindestens einer Quellenposition mindestens einer radioaktiven Strahlungsquelle geleitet wird.

15. Ein Computerprogramm gemäß Anspruch 14, das zudem ein System gemäß einem der Ansprüche 1 bis 12 steuert.

## Revendications

1. Système de traitement de brachythérapie comprenant au moins un tube de guidage pour guider des sources de rayonnement radioactif dans un patient ; au moins un dispositif de mesure guidé par ledit au moins un tube de guidage par rapport à au moins une position de source d'au moins une source de rayonnement radioactif ;
une unité d'entrée (100) conçue pour recevoir des données de suivi comprenant une pluralité de valeurs de suivi acquises en une pluralité de positions le long dudit au moins un tube de guidage ;
une unité de reconstruction (200) conçue pour obtenir une reconstruction dudit au moins un tube de guidage en fonction des données de suivi ;
ledit système étant **caractérisé en ce qu'**il comprend en outre :
une unité de détermination (300) conçue pour déterminer,
en fonction de la reconstruction, au moins une position de mesure de l'au moins un dispositif de mesure.

2. Système selon la revendication 1, dans lequel les données de suivi sont acquises à l'aide d'un suivi électromagnétique et/ou par fibre optique.

3. Système selon la revendication 1, comprenant en outre
une unité de calcul conçue pour recevoir des données de position de mesure indiquant l'au moins une position de mesure par rapport à l'au moins une
position source, et
pour dériver,
en fonction d'un plan de thérapie, des données de dose prédite pour l'au moins
une position de mesure.

4. Système selon la revendication 3, comprenant en outre
une unité de vérification conçue pour dériver,
en fonction des données de rayonnement acquises par l'au moins un dispositif de mesure à l'au moins une position de mesure, des données de dose délivrée de la dose délivrée par l'au moins une source de rayonnement radioactif à l'au moins une position de mesure, et
pour comparer
lesdites données de dose délivrée aux données de dose prédite dérivées
pour ladite position de mesure particulière.

5. Système selon la revendication 4, comprenant en outre
une unité d'affichage conçue pour générer
une première représentation graphique des données de dose délivrée et une seconde représentation graphique des données de dose prédite, et
pour afficher conjointement la première représentation graphique et la seconde représentation graphique.

6. Système selon la revendication 4, comprenant en outre
une unité d'indication conçue pour déclencher
au moins une indication lorsque les données de dose délivrée
et les données de dose prédite s'écartent les unes des autres d'une tolérance prédéfinie.

7. Système selon la revendication 3, comprenant en outre
une unité d'optimisation conçue pour optimiser l'au moins une position de mesure en
au moins une position de mesure optimisée en fonction des données de dose prédite prédites en fonction du plan de thérapie.

8. Système selon la revendication 7, dans lequel l'unité d'optimisation est en outre conçue pour optimiser une séquence d'insertion d'une pluralité de tubes de guidage en fonction des données de distribution de dose prédite prédites en fonction d'un plan de thérapie.

9. Système selon la revendication 7, l'unité d'optimisation étant en outre conçue pour effectuer l'optimisation de l'au moins une
position de mesure de l'au moins un dispositif de mesure vers l'au moins une position de mesure optimisée comprenant les étapes suivantes :
la détermination d'au moins une autre position de mesure d'au moins un autre dispositif de mesure ;
l'optimisation de l'au moins une position de mesure de l'au moins un dispositif de mesure vers l'au moins une position de mesure optimisée en fonction de l'au moins une autre position de mesure de l'au moins un autre dispositif de mesure ; et/ou
l'optimisation de l'au moins une autre position de mesure de l'au moins un autre dispositif de mesure vers au moins une autre position de mesure optimisée en fonction de l'au moins une position de mesure de l'au moins un dispositif de mesure.

10. Système selon la revendication 7, dans lequel l'unité d'optimisation est en outre conçue pour effectuer l'optimisation de l'au moins une position de mesure vers l'au moins une position de mesure optimisée comprenant les étapes suivantes:
l'identification d'une pluralité de positions candidates pour le dispositif de mesure;
la prédiction, en fonction du plan de thérapie, des données de dose prédite pour chaque position de la pluralité de positions de mesure candidates; et
la détermination, en fonction de la pluralité de positions de mesure candidates, d'au moins une position de mesure optimisée par application d'un procédé de pénalité aux données de dose prédite.

11. Système selon la revendication 10, dans lequel l'application du procédé de pénalité aux données de dose prédite comprend l'application d'une pluralité de fonctions de pénalité et la réunion de la pluralité de fonctions de pénalité en une fonction objective à l'aide d'une somme pondérée.

12. Système selon la revendication 7, dans lequel l'unité d'optimisation est en outre conçue pour recevoir
au moins une indication de changement d'un changement dans le plan de thérapie; et
pour ajuster
l'optimisation de l'au moins une position de mesure à l'au moins
une position de mesure optimisée en fonction de l'au moins une indication de changement.

13. Procédé d'assurance de qualité lors d'un traitement de brachythérapie, ledit procédé comprenant les étapes suivantes:
la réception des données de suivi comprenant une pluralité de valeurs de suivi acquises en une pluralité de positions le long d'au moins un tube de guidage ;
l'obtention d'une reconstruction dudit au moins un tube de guidage en fonction des données de suivi ; et
la détermination, en fonction de la reconstruction, d'au moins une position de mesure d'au moins un dispositif de mesure guidé par ledit au moins un tube de guidage par rapport à au moins une position de source d'au moins une source de rayonnement radioactif.

14. Programme informatique, lequel, lorsqu'il est exécuté par une unité de traitement, est conçu pour mettre en œuvre un procédé comprenant les étapes suivantes:
la réception des données de suivi comprenant une pluralité de valeurs de suivi acquises en une pluralité de positions le long d'au moins un tube de guidage;
l'obtention d'une reconstruction dudit au moins un tube de guidage en fonction des données de suivi; et
la détermination, en fonction de la reconstruction, d'au moins une position de mesure d'au moins un dispositif de mesure guidé par ledit au moins un tube de guidage par rapport à au moins une position de source d'au moins une source de rayonnement radioactif.

15. Programme informatique selon la revendication 14, configuré en outre pour commander un système selon l'une quelconque des revendications 1 à 12.
